# EUROPEAN PATENT SPECIFICATION

(11) **EP 3 313 311 B1**
(45) Date of publication and mention of the grant of the patent: **07.08.2019**
(21) Application number: 16741439.0
(22) Date of filing: 28.06.2016
(51) Int. Cl.: A61B 18/14

(54) **OPEN-IRRIGATED ABLATION CATHETER**
OFFENER BENETZTER ABLATIONSKATHETER
CATHÉTHER D'ABLATION OUVERT IRRIGUÉ

(30) Priority: 29.06.2015 US 201562186359 P
(43) Date of publication of application: 02.05.2018
(73) Proprietor: Boston Scientific Scimed Inc., Maple Grove, Minnesota 55311 (US)
(72) Inventor: MIRIGIAN, Mark D., San Jose, California 95138 (US); KIM, Isaac J., San Jose, California 95135 (US); FORREST, Mark D., Sunnyvale, California 94085 (US); CHEUNG, Desmond, San Jose, California 95112 (US); HARVEY, Guy R., Milpitas, California 95035-8646 (US); KURSE, Ravi, Fremont, California 94536 (US); ORTIZ, Ofelia, San Jose, California 95122 (US); VELILLA, Simplicio A., Santa Clara, California 95051 (US); NGUYEN, Oanh, San Jose, California 95121 (US); WYLIE, Bryan, Redwood City, California 94062 (US); ESHEIM, Taiki, Morgan Hill, California 95037 (US)
(74) Representative: Pfenning, Meinig & Partner mbB
(86) International application number: PCT/US2016/039915
(87) International publication number: WO 2017/004093

(56) References cited:
- EP-A1- 2 380 519
- US-A1- 2010 331 658
- US-A1- 2014 171 936
- US-A1- 2014 276 759

## Description

### TECHNICAL FIELD

The present disclosure relates to medical devices. More specifically, the invention relates to devices and systems for performing ablation and mapping functions.

### BACKGROUND

Aberrant conductive pathways disrupt the normal path of the heart's electrical impulses. For example, conduction blocks can cause the electrical impulse to degenerate into several circular wavelets that disrupt the normal activation of the atria or ventricles. The aberrant conductive pathways create abnormal, irregular, and sometimes life-threatening heart rhythms called arrhythmias. Ablation is one way of treating arrhythmias and restoring normal conduction. The sources of the aberrant pathways (called focal arrhythmia substrates) are located or mapped using mapping electrodes situated in a desired location. After mapping, the physician may ablate the aberrant tissue. In radio frequency (RF) ablation, RF energy is directed from the ablation electrode through tissue to an electrode to ablate the tissue and form a lesion.

US 2014/276759A1 discloses an open-irrigated ablation catheter that may include a catheter body, an electrode tip body with one or more irrigation ports at a distal end, wherein the irrigation ports may have a diameter approximately within a range of 0.0254 cm to 0.0508 cm (0.01 in to 0.02 in), and a proximal insert for providing cooling fluid to a proximal end of the electrode tip body. The proximal end of the electrode tip body may additionally have one or more openings that allow cooling fluid to exit and cool the proximal region of the electrode tip body.

US 2010/331658A1 discloses an open-irrigated catheter system that comprises a tip section, a distal insert, and mapping electrodes. The tip section has an exterior wall that defines an open interior region within the tip section. The exterior wall includes mapping electrode openings and irrigation ports. The exterior wall is conductive for delivering radio frequency (RF) energy for an RF ablation procedure. The irrigation ports are in fluid communication with the open interior region to allow fluid to flow from the open interior region through the irrigation ports. The distal insert is positioned within the tip section to separate the open region into a distal fluid reservoir and a proximal fluid reservoir. The mapping electrodes are positioned in the mapping electrode openings in the tip section.

EP 2380519A1 discloses a catheter that carries a position sensor in a distal, on-axis position in an irrigated ablation tip electrode. The tip electrode has a shell wall that defines a cavity through which fluid flows and exits via fluid ports formed in the shell wall. The cavity is sealed by an internal member extends into the cavity with a baffle portion and a distal portion. The distal portion safely houses the position sensor and the baffle portion diffuses and disperses fluid entering the tip electrode for a more uniform flow through the cavity. The distal portion is configured to provide an annular region that runs along the length of the tip electrode to better feed fluid to the more distal fluid ports on the tip electrode for more uniform cooling at all locations on the tip electrode.

US 2014/171936A1 discloses an apparatus, consisting of an insertion tube having a distal end configured for insertion into proximity with tissue in a body of a patient and containing a lumen having an electrical conductor for conveying electrical energy to the tissue. The apparatus further includes a conductive cap attached to the distal end of the insertion tube and coupled electrically to the electrical conductor, wherein the conductive cap has an outer surface. In addition there are a multiplicity of optical fibers contained within the insertion tube, each fiber terminating in proximity to the outer surface of the cap, and being configured to convey optical radiation to and from the tissue while the electrical energy is being conveyed to the tissue.

### SUMMARY

In an Example 1, an open-irrigated catheter system comprises a catheter body; a tip assembly, coupled to a distal end of the catheter body, and having an exterior wall that defines an interior region within the tip assembly, wherein the exterior wall includes a plurality of proximal irrigation ports and a plurality of distal irrigation ports, and wherein the exterior wall is conductive for delivering radio frequency (RF) energy for an RF ablation procedure; at least one fluid chamber defined within the interior region, wherein the at least one fluid chamber is in fluid communication with at least one of the plurality of proximal irrigation ports and the plurality of distal irrigation ports; and at least one fluid lumen extending from a fluid source, through the catheter body, to the tip assembly, wherein the at least one fluid lumen is in fluid communication with the at least one fluid chamber.
In an Example 2, the system of Example 1, wherein the plurality of proximal irrigation ports comprises at least 12 irrigation ports.
In an Example 3, the system of Example 2, wherein the plurality of proximal irrigation ports comprises at least 36 irrigation ports.
In an Example 4, the system of any of Examples 1-3, wherein the plurality of proximal irrigation ports is arranged in an evenly-spaced array positioned circumferentially around a proximal portion of the tip assembly.
In an Example 5, the system of Example 4, wherein the array comprises at least one row.
In an Example 6, the system of Example 5, the array comprising a first row of irrigation ports and a second row of irrigation ports, wherein the second row is aligned with the first row to form a plurality of pairs of longitudinally-aligned irrigation ports.
In an Example 7, the system of Example 5, the array comprising a first row of irrigation ports and a second row of irrigation ports, wherein the second row is offset from the first row.
In an Example 8, the system of any of Examples 1-7, wherein the at least one fluid chamber comprises a proximal fluid chamber and a distal fluid chamber, wherein the proximal fluid chamber is in fluid communication with the plurality of proximal irrigation ports, and wherein the distal fluid chamber is in fluid communication with the plurality of distal irrigation ports.
In an Example 9, the system of Example 8, wherein the at least one fluid lumen comprises a first fluid lumen and a second fluid lumen, wherein the first fluid lumen is in fluid communication with the proximal fluid chamber and wherein the second fluid lumen is in fluid communication with the distal fluid chamber.
In an Example 10, the system of Example 9, wherein the fluid source provides a first stream of fluid to the proximal fluid chamber and a second stream of fluid to the distal fluid chamber, wherein the second stream of fluid is provided at a greater flow rate than the first stream of fluid.
In an Example 11, the system of Example 8, wherein the at least one fluid lumen comprises a single fluid lumen that is in fluid communication with the proximal fluid chamber, the system further comprising a fluid path extending between the proximal fluid chamber and the distal fluid chamber such that the proximal fluid chamber is in fluid communication with the distal fluid chamber.
In an Example 12, the system of Example 11, further comprising a distal insert, wherein the fluid path is defined within the distal insert.
In an Example 13, the system of any of Examples 1-12, wherein the external wall includes a plurality of mapping-electrode openings, and wherein the system further comprises a plurality of mapping electrodes, wherein each of the plurality of mapping electrodes is positioned within one of the plurality of mapping-electrode openings.
In an Example 14, the system of any of Examples 1-13, wherein the plurality of distal irrigation ports comprises six distal irrigation ports, wherein the six distal irrigation ports are evenly-spaced circumferentially around a distal portion of the tip assembly.
In an Example 15, the system of any of Examples 1-14, wherein each of the proximal irrigation ports includes a diameter of between 0.00254 cm (0.001 in.) and 0.01016 cm (0.004 in.).
In an Example 16, an open-irrigated catheter system comprises a catheter body; a tip assembly, coupled to a distal end of the catheter body, and having an exterior wall that defines an interior region within the tip assembly, wherein the exterior wall includes a plurality of proximal irrigation ports and a plurality of distal irrigation ports, and wherein the exterior wall is conductive for delivering radio frequency (RF) energy for an RF ablation procedure; at least one fluid chamber defined within the interior region, wherein the at least one fluid chamber is in fluid communication with at least one of the plurality of proximal irrigation ports and the plurality of distal irrigation ports; and at least one fluid lumen extending from a fluid source, through the catheter body, to the tip assembly, wherein the at least one fluid lumen is in fluid communication with the at least one fluid chamber.
In an Example 17, the system of Example 16, wherein the plurality of proximal irrigation ports comprises at least 12 irrigation ports.
In an Example 18, the system of Example 17, wherein the plurality of proximal irrigation ports comprises at least 36 irrigation ports.
In an Example 19, the system of Example 16, wherein the plurality of proximal irrigation ports is arranged in an evenly-spaced array positioned circumferentially around a proximal portion of the tip assembly.
In an Example 20, the system of Example 19, wherein the array comprises at least one row.
In an Example 21, the system of Example 20, the array comprising a first row of irrigation ports and a second row of irrigation ports, wherein the second row is aligned with the first row to form a plurality of pairs of longitudinally-aligned irrigation ports.
In an Example 22, the system of Example 20, the array comprising a first row of irrigation ports and a second row of irrigation ports, wherein the second row is offset from the first row.
In an Example 23, the system of Example 16, wherein the at least one fluid chamber comprises a proximal fluid chamber and a distal fluid chamber, wherein the proximal fluid chamber is in fluid communication with the plurality of proximal irrigation ports, and wherein the distal fluid chamber is in fluid communication with the plurality of distal irrigation ports.
In an Example 24, the system of Example 23, wherein the at least one fluid lumen comprises a first fluid lumen and a second fluid lumen, wherein the first fluid lumen is in fluid communication with the proximal fluid chamber and wherein the second fluid lumen is in fluid communication with the distal fluid chamber.
In an Example 25, the system of Example 24, wherein the fluid source provides a first stream of fluid to the proximal fluid chamber and a second stream of fluid to the distal fluid chamber, wherein the second stream of fluid is provided at a greater flow rate than the first stream of fluid.
In an Example 26, the system of Example 23, wherein the at least one fluid lumen comprises a single fluid lumen that is in fluid communication with the proximal fluid chamber, the system further comprising a fluid path extending between the proximal fluid chamber and the distal fluid chamber such that the proximal fluid chamber is in fluid communication with the distal fluid chamber.
In an Example 27, the system of Example 26, further comprising a distal insert, wherein the fluid path is defined within the distal insert.
In an Example 28, the system of Example 16, wherein the external wall includes a plurality of mapping-electrode openings, and wherein the system further comprises a plurality of mapping electrodes, wherein each of the plurality of mapping electrodes is positioned within one of the plurality of mapping-electrode openings.
In an Example 29, the system of Example 16, wherein the plurality of distal irrigation ports comprises six distal irrigation ports, wherein the six distal irrigation ports are evenly-spaced circumferentially around a distal portion of the tip assembly.
In an Example 30, the system of Example 16, wherein each of the proximal irrigation ports includes a diameter of between 0.00254 cm (0.001 in.) and 0.01016 cm (0.004 in.).
In an Example 31, an open-irrigated catheter comprises a tip assembly having an exterior wall that defines an interior region within the tip assembly, wherein the exterior wall includes a plurality of proximal irrigation ports and a plurality of distal irrigation ports, and wherein the exterior wall is conductive for delivering radio frequency (RF) energy for an RF ablation procedure; at least one fluid chamber defined within the interior region, wherein the at least one fluid chamber is in fluid communication with at least one of the plurality of proximal irrigation ports and the plurality of distal irrigation ports; and at least one fluid lumen extending from a fluid source, through the catheter body, to the tip assembly, wherein the at least one fluid lumen is in fluid communication with the at least one fluid chamber.
In an Example 32, the catheter of Example 31, wherein the at least one fluid chamber comprises a proximal fluid chamber and a distal fluid chamber, wherein the proximal fluid chamber is in fluid communication with the plurality of proximal irrigation ports, and wherein the distal fluid chamber is in fluid communication with the plurality of distal irrigation ports.
In an Example 33, the catheter of Example 32, wherein the at least one fluid lumen comprises a first fluid lumen and a second fluid lumen, wherein the first fluid lumen is in fluid communication with the proximal fluid chamber and wherein the second fluid lumen is in fluid communication with the distal fluid chamber.
In an Example 34, the catheter of Example 33, wherein the fluid source provides a first stream of fluid to the proximal fluid chamber and a second stream of fluid to the distal fluid chamber, wherein the second stream of fluid is provided at a greater flow rate than the first stream of fluid.
In an Example 35, the catheter of Example 31, wherein the plurality of distal irrigation ports comprises six distal irrigation ports, wherein the six distal irrigation ports are evenly-spaced circumferentially around a distal portion of the tip assembly.

While multiple embodiments are disclosed, still other embodiments of the present invention will become apparent to those skilled in the art from the following detailed description, which shows and describes illustrative embodiments of the invention. Accordingly, the drawings and detailed description are to be regarded as illustrative in nature and not restrictive.

### BRIEF DESCRIPTION OF THE DRAWINGS

FIG. 1 depicts an illustrative mapping and ablation system that includes an open-irrigated catheter in accordance with embodiments of the invention.
FIGS. 2A-2D depict an illustrative tip assembly for a mapping and ablation catheter in accordance with embodiments of the invention.
FIGS. 3A-3B depict an illustrative tip assembly for a mapping and ablation catheter in accordance with embodiments of the invention.
FIG. 4 depicts an illustrative tip assembly for a mapping and ablation catheter in accordance with embodiments of the invention.
FIG. 5 depicts an illustrative tip assembly for a mapping and ablation catheter in accordance with embodiments of the invention.
FIG. 6 depicts an illustrative tip assembly for a mapping and ablation catheter in accordance with embodiments of the invention.

While the invention is amenable to various modifications and alternative forms, specific embodiments have been shown by way of example in the drawings and are described in detail below. The intention, however, is not to limit the invention to the particular embodiments described. On the contrary, the invention is intended to cover all modifications, equivalents, and alternatives falling within the scope of the invention as defined by the appended claims.

### DETAILED DESCRIPTION

Embodiments of the disclosure relate to a radiofrequency (RF) ablation catheter system. In embodiments, the catheter may be a hybrid catheter, which may be configured to be used for both localized mapping and ablation functions. The hybrid catheter may be configured to provide localized, high resolution ECG signals during ablation. This localized mapping may enable the ablation procedure to be more precise than that which can be achieved with conventional, non-hybrid ablation catheters. The catheter has an open-irrigated catheter design. A cooling fluid, such as a saline, is delivered through the catheter to a tip assembly having a tissue ablation electrode, where the fluid exits through irrigation ports defined in the tissue ablation electrode to cool the electrode and surrounding tissue during ablation. Clinical benefits of such a catheter may include, but are not limited to, controlling the temperature and reducing coagulum formation on the tip of the catheter, preventing impedance rise of tissue in contact with the catheter tip, and maximizing potential energy transfer to the tissue. Additionally, the localized intra cardiac electrical activity can be recorded in real time or near-real time at the location of energy delivery.

A number of adverse effects may be encountered with open-irrigated RF ablation catheters and may include, for example, excessive heating of a proximal portion of the tissue ablation electrode (e.g., due to edge effect), current density concentrations (e.g., due to geometric discontinuities, radius changes, etc.), and/or the like. Some developments to address these issues have included the addition of proximal irrigation ports similar in size to the distal irrigation ports, and the replacement of larger distal irrigation ports with a large number of very small irrigation ports dispersed throughout the ablation electrode. Both of these solutions may provide some mitigation of proximal heating due to edge effect, but have a tendency to change the current pathway due to the cloud of cooling fluid that forms around the electrode. This can either result in current loss, as the current shunts through the ionic fluid and away from the target tissue, or result in excessive tissue heating, which may be a byproduct of a localized saline cloud driving too much current into the tissue.

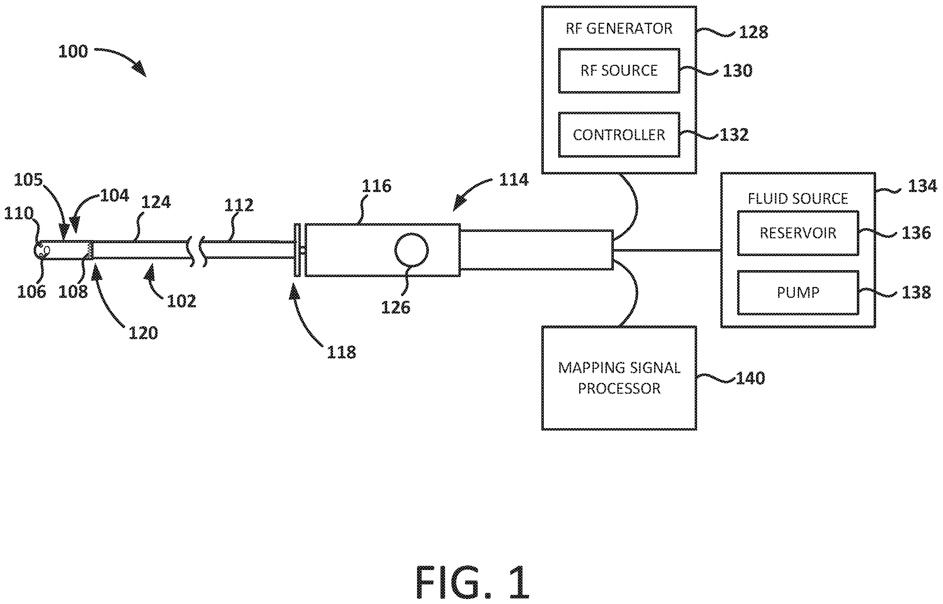

Embodiments of the invention provide an open-irrigated catheter design that includes distal irrigation ports and much smaller proximal irrigation ports (that may be referred to as "micro-holes"), thereby providing cooling fluid flow to cool the proximal portion of the electrode, while maintaining the desired current pathways due to the more forceful flow of fluid from the larger distal irrigation ports. FIG. 1 depicts a mapping and ablation system 100 that includes an open-irrigated ablation catheter 102, according to embodiments of the invention. The illustrated catheter 102 includes a tip assembly 104 having a tissue ablation electrode 105, with mapping microelectrodes 106, proximal irrigation ports 108, and distal irrigation ports 110. The catheter 102 includes a catheter body 112 and a proximal catheter handle assembly 114, having a handle 116, coupled to a proximal end 118 of the catheter body 112. The tip assembly 104 is coupled to a distal end 120 of the catheter body 112.

In some instances, the mapping and ablation system 100 may be utilized in ablation procedures on a patient and/or in ablation procedures on other objects. In various embodiments, the ablation catheter 102 may be configured to be introduced into or through the vasculature of a patient and/or into or through any other lumen or cavity. In an example, the ablation catheter 102 may be inserted through the vasculature of the patient and into one or more chambers of the patient's heart (e.g., a target area). When in the patient's vasculature or heart, the ablation catheter 102 may be used to map and/or ablate myocardial tissue using the microelectrodes 106 and/or the tissue ablation electrode 105. In embodiments, the tissue ablation electrode 105 may be configured to apply ablation energy to myocardial tissue of the heart of a patient.

According to embodiments, the tissue ablation electrode 105 may be, or be similar to, any number of different tissue ablation electrodes such as, for example, the IntellaTip MiFi,™ or the Blazer™ Ablation tip, both of which are available from Boston Scientific of Marlborough, Massachusetts. In embodiments, the tissue ablation electrode 105 may have any number of different sizes, shapes, and/or other configuration characteristics. The tissue ablation electrode 105 may be any length and may have any number of the microelectrodes 106 positioned therein and spaced circumferentially and/or longitudinally about the tissue ablation electrode 105. In some instances, the tissue ablation electrode 105 may have a length of between one (1) mm and twenty (20) mm, three (3) mm and seventeen (17) mm, or six (6) mm and fourteen (14) mm. In one illustrative example, the tissue ablation electrode 105 may have an axial length of about eight (8) mm. In another illustrative example, the tissue ablation electrode 105 may include an overall length of approximately 4-10mm. In embodiments, the tissue ablation electrode 105 may include an overall length of approximately 4 mm, 4.5 mm, and/or any other desirable length. In some cases, the plurality of microelectrodes 106 may be spaced at any interval about the circumference of the tissue ablation electrode 105. In one example, the tissue ablation electrode 105 may include at least three microelectrodes 106 equally or otherwise spaced about the circumference of the tissue ablation electrode 105 and at the same or different longitudinal positions along the longitudinal axis of the tissue ablation electrode 105.

In embodiments, the catheter 102 may include a deflectable catheter region 124 configured to allow the catheter 102 to be steered through the vasculature of a patient, and which may enable the tissue ablation electrode 105 to be accurately placed adjacent a targeted tissue region. A steering wire (not shown) may be slidably disposed within the catheter body 112. The handle assembly 114 may include one or more steering members 126 such as, for example, rotating steering knobs that are rotatably mounted to the handle 116. Rotational movement of a steering knob 126 relative to the handle 116 in a first direction may cause a steering wire to move proximally relative to the catheter body 112 which, in turn, tensions the steering wire, thus pulling and bending the catheter deflectable region 124 into an arc; and rotational movement of the steering knob 126 relative to the handle 116 in a second direction may cause the steering wire to move distally relative to the catheter body 112 which, in turn, relaxes the steering wire, thus allowing the catheter 102 to return toward its original form. To assist in the deflection of the catheter 102, the deflectable catheter region 124 may be made of a lower durometer plastic than the remainder of the catheter body 112.

According to embodiments, the catheter body 112 includes one or more cooling fluid lumens (not shown) and may include other tubular element(s) to provide desired functionality to the catheter 102. The addition of metal in the form of a braided mesh layer sandwiched in between layers of plastic tubing may be used to increase the rotational stiffness of the catheter 102.

The illustrated system 100 includes an RF generator 128 used to generate RF energy for use during an ablation procedure. The RF generator 128 may include an RF source 130 that produces the RF energy and a controller 132 for controlling the timing, level, and/or other characteristics of the RF energy delivered through the tip assembly 104. The RF generator 128 may be configured to deliver ablation energy to the ablation catheter 102 in a controlled manner in order to ablate the target tissue sites. Ablation of tissue within the heart is well known in the art, and thus for purposes of brevity, the RF generator 128 will not be described in further detail. Further details regarding RF generators are provided in U.S. Patent 5,383,874.

The illustrated system 100 also includes a fluid source 134, having a fluid reservoir 136 and a pump 138 for providing cooling fluid, such as a saline, through the catheter 102 and out through the irrigation ports 108 and 110. A mapping signal processor 140 may be connected to the electrodes 106, also referred to herein as microelectrodes. The mapping signal processor 140 and electrodes 106 may be configured to detect electrical activity of the heart. This electrical activity may be evaluated to analyze an arrhythmia and to determine where to deliver the ablation energy as a therapy for the arrhythmia. Although the mapping processor 140 and RF generator 128 are shown as discrete components, they can alternatively be incorporated into a single integrated device.

One of ordinary skill in the art will understand that various components such as, for example, aspects of the RF generator 128, the fluid source, 134, and/or the mapping signal processor 140, may be implemented using software, hardware, and/or firmware. Various methods of operation may be implemented as a set of instructions contained on a computer-accessible medium capable of directing a processor to perform the respective method.

The RF ablation catheter 102 as described may be used to perform various diagnostic functions to assist the physician in an ablation treatment. For example, in some embodiments, the catheter 102 may be used to ablate cardiac arrhythmias, and at the same time provide real-time assessment of a lesion formed during RF ablation. Real-time assessment of the lesion may involve any of monitoring surface and/or tissue temperature at or around the lesion, reduction in the electrocardiogram signal, a drop in impedance, direct and/or surface visualization of the lesion site, and imaging of the tissue site (e.g., using computed tomography, magnetic resonance imaging, ultrasound, etc.). In addition, the presence of the microelectrodes within the RF tip electrode can operate to assist the physician in locating and positioning the tip electrode at the desired treatment site, and to determine the position and orientation of the tip electrode relative to the tissue to be ablated.

Illustrative catheters that may be used as the catheter 102 may include, among other ablation and/or mapping catheters, those described in U.S. Patent Publication Number 2008-0243214 A1 filed on March 26, 2008, and entitled HIGH RESOLUTION ELECTROPHYSIOLOGY CATHETER, and U.S. Patent 8,414,579 filed on June 23, 2010, entitled MAP AND ABLATE OPEN IRRIGATED HYBRID CATHETER. Alternatively, or in addition, catheters that may be used as the catheter 102 may include, among other ablation and/or mapping catheters, those described in U.S. Patent 5,647,870 filed on January 16, 1996, as a continuation of U.S. Serial No. 206,414, filed March 4, 1994 as a continuation-in-part of U.S. Serial Number 33,640, filed March 16, 1993, entitled MULTIPLE ELECTRODE SUPPORT STRUCTURES, U.S. Patent 6,647,281 filed on April 6, 2001, entitled EXPANDABLE DIAGNOSTIC OR THERAPEUTIC APPARATUS AND SYSTEM FOR INTRODUCING THE SAME INTO THE BODY, and U.S. Patent No. 8,128,617 filed on May 27, 2008, entitled ELECTRICAL MAPPING AND CRYO ABLATING WITH A BALLOON CATHETER.

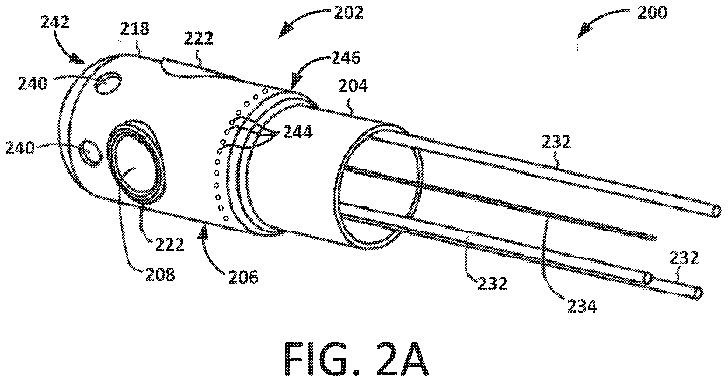

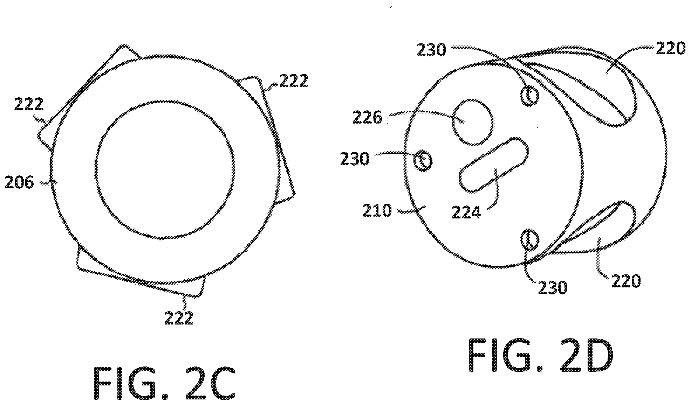

FIGS. 2A - 2D illustrate a hybrid catheter 200, according to embodiments of the invention, having proximal and distal irrigation ports and three microelectrodes used to perform a mapping function. The illustrated catheter 200 includes a tip assembly 202, having a tip body 204, and an open-irrigated ablation electrode 206 used to perform mapping and ablation functions. In embodiments, the ablation functions may be performed, in part, by the ablation electrode 206, which may function as an RF electrode. The mapping functions may be performed, at least in part, by mapping electrodes 208.

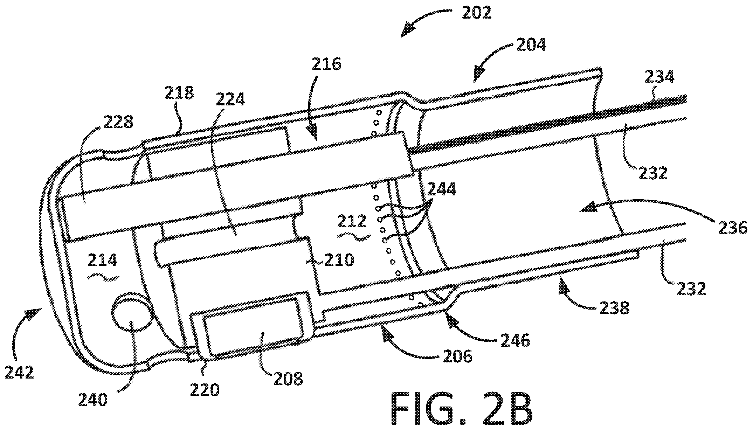

With particular reference to FIG. 2B, the illustrated tip assembly 202 includes a generally hollow ablation electrode 206 having a distal insert 210 disposed therein and configured to separate a proximal fluid chamber 212 and distal fluid chamber 214. The tip assembly 202 has an open interior region 216 defined by an exterior wall 218 of the tip assembly 202. Fluid flow through the chambers 212 and 214 may be used to provide internal, targeted cooling of portions of the ablation electrode 206. In the illustrated embodiments, the hollow tip body 204 has a generally cylindrical shape, but in other embodiments, the tip body 204 may have any number of different shapes such as, for example, an elliptical shape, a polygonal shape, and/or the like. By way of an example and not limitation, embodiments of the tip assembly 202 may have a diameter on the order of about 0.2032 cm to 0.254 cm (0.08 in to 0.1 in), a length on the order of about 0.508 cm to 0.762 cm (0.2 in to 0.3 in), and an exterior wall 218 with a thickness on the order of about 0.00762 cm to 0.01016 cm (0.003 in to 0.004 in).

As the terms are used herein with respect to ranges of measurements (such as those disclosed immediately above), "about" and "approximately" may be used, interchangeably, to refer to a measurement that includes the stated measurement and that also includes any measurements that are reasonably close to the stated measurement, but that may differ by a reasonably small amount such as will be understood, and readily ascertained, by individuals having ordinary skill in the relevant arts to be attributable to measurement error, differences in measurement and/or manufacturing equipment calibration, human error in reading and/or setting measurements, adjustments made to optimize performance and/or structural parameters in view of differences in measurements associated with other components, particular implementation scenarios, and/or the like.

According to embodiments, the distal insert 210 may be made of plastic components such as, for example, Ultem. Various distal insert embodiments include design elements configured for self-positioning the distal insert during manufacturing. Such embodiments may facilitate reducing the number of processing steps to join the distal insert to the tip electrode. Additionally, various distal insert embodiments may be configured for self-alignment and configured to isolate electrical components from the irrigation fluid. Some embodiments are configured for self-alignment, some embodiments are configured to isolate electrical components from the irrigation fluid, and some embodiments are configured for both self-alignment and for isolating electrical components from the irrigation fluid. Various designs of distal inserts as described above are described in U.S. Patent 8,414,579.

In embodiments, the ends of the distal insert 210 may be encapsulated with adhesives to provide a seal between the proximal and distal chambers 212 and 214. In embodiments, the distal insert 210 may include openings or apertures 220, each opening 220 sized to receive a microelectrode 208 and a corresponding noise artifact isolator 222. These microelectrodes 208 may be used to image localized intracardiac activity. The microelectrodes 208 may, for example, be used to record high resolution, precise localized electrical activity, to prevent excessive heating of the ablation electrode 206, to allow greater delivery of power, to prevent the formation of coagulum and to provide the ability to diagnose complex ECG activity. In embodiments, the microelectrodes 208 are small, independent diagnostic sensing electrodes embedded within the walls of the ablation electrode 206 of the RF ablation catheter 200. The noise artifact isolator 222 electrically isolates the small electrodes 208 from the conductive exterior wall 218 of the ablation electrode 206. According to embodiments, the noise artifact isolator 222 may be a polymer-based material sleeve and/or adhesive that encapsulates the microelectrodes 208. The isolator 222 isolates the noise entrance creating a much cleaner electrogram during an RF ablation mode. These electrically-isolated microelectrodes 208 are able to sense highly localized electrical activity, avoid a far-field component, and simultaneously achieve the ability to ablate tissue without noise artifact during RF mode.

The illustrated distal insert 210 also includes a fluid conduit or passage 224 to permit fluid to flow from the proximal fluid reservoir 212 to the distal fluid reservoir 214, a thermocouple opening 226 sized to receive a thermocouple 228, and openings 230 sized to receive electrical conductors 232 used to provide electrical connections to the microelectrodes 208. Also illustrated is a thermocouple wire 234 connected to the thermocouple 228. According to embodiments, the distal insert 210 may be fabricated from stainless steel, a polymer, and/or the like. In embodiments, a proximal insert (not shown) may be disposed in an interior region 236 of a proximal portion 238 of the tip body 204. The proximal insert may, in embodiments, prevent fluid from flowing back out of the proximal fluid chamber 212, and may include apertures for the wires, conductors, and one or more fluid conduits.

According to embodiments, the ablation electrode 206 may be formed from a conductive material. For example, some embodiments use a platinum-iridium alloy. Some embodiments use an alloy with approximately 90% platinum and 10% iridium. The conductive material of the ablation electrode 206 is used to conduct RF energy used to form legions during the ablation procedure. In embodiments, the ablation electrode 206 includes a plurality of distal irrigation ports 240 near the distal end 242 of the ablation electrode 206, and a plurality of proximal irrigation ports 244 near the proximal end 246 of the ablation electrode 206. By way of example and not limitation, in embodiments, the distal irrigation ports 240 may each have a diameter approximately within a range of 0.0254 cm to 0.0508 cm (0.01 in to 0.02 in). Fluid, such as a saline solution, flows from the distal fluid reservoir 214, through these ports 240, to the exterior of the catheter 200. This fluid is used to cool the ablation electrode 206 and the tissue near the electrode 206. This temperature control may facilitate reduction of coagulum formation on the tip of the catheter 200, prevents impedance rise of tissue in contact with the catheter tip, and increases energy transfer to the tissue because of the lower tissue impedance.

According to embodiments, the proximal irrigation ports 244 are configured to facilitate a fluid flow out of the ablation electrode 206 to minimize char formation on the proximal region of the ablation electrode 206. Providing proximal irrigation ports may also facilitate minimizing risk of thrombus and the potential for emboli. According to embodiments, the proximal irrigation ports are relatively small as compared, for example, to the distal irrigation ports. For example, in embodiments, each of the proximal irrigation ports 244 may have a diameter of approximately 0.00254 cm (0.001 in.) to 0.01016 cm (0.004 in.). In this manner, conventional flow characteristics associated with distal irrigation ports may be maintained, so as to maintain effective RF conduction for ablation. That is, for example, the proximal irrigation ports may be configured to provide a flow rate sufficient for achieving desired cooling results external to the ablation electrode 206, while maintaining desired flow characteristics from the distal irrigation ports. The arrangement, size, and/or number of proximal irrigation ports may be adjusted based on the characteristics of the distal irrigation ports, the fluid chambers, the tip, and/or the like. In embodiments, the catheter 200 may include 6 distal irrigation ports 240 and between 12 and 36 proximal irrigation ports 244. In other embodiments, the catheter may include more than 36 proximal irrigation ports 244 such as, for example, 54 ports, 72 ports, and/or the like.

Various embodiments isolate the microelectrode signal wires from the cooling fluid circulating in the proximal chamber of the hollow ablation electrode, and thus are expected to reduce the noise that is contributed form the internal cooling fluid circulation. The fluid seal can be provided without bonding or adhesive. The electrical components within the tip are isolated form the cooling flow of irrigation fluid while the irrigation fluid maintains internal cooling of the proximal and distal portions of the tip electrode. Further, such designs may have the potential of increasing the accuracy of the temperature readings from the thermocouple, and are described in U.S. Patent 8,414,579, mentioned above.

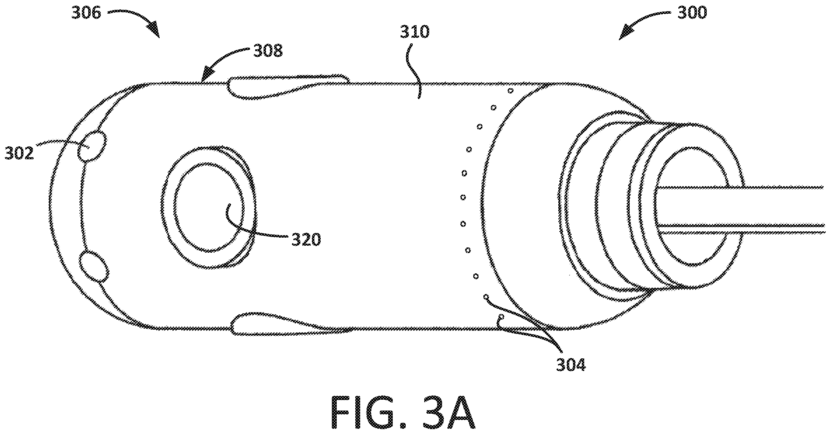

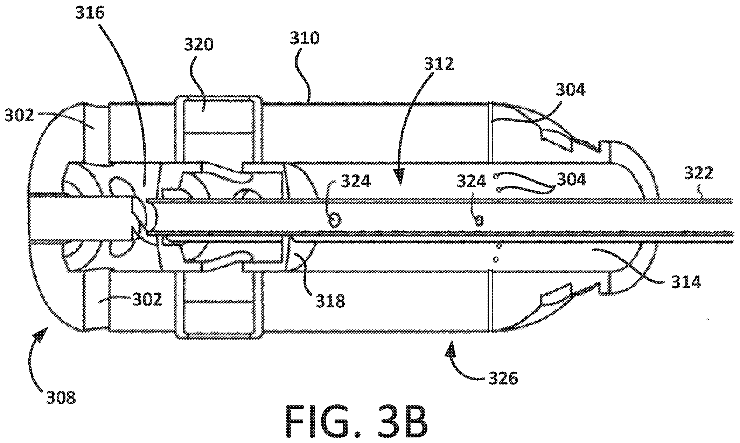

FIGS. 3A and 3B illustrate a hybrid catheter 300, according to embodiments of the invention, having a plurality of distal irrigation ports 302 and a plurality of proximal irrigation ports 304. The hybrid catheter 300 includes a tip assembly 306, having an ablation electrode 308. The ablation electrode 308 includes an external wall 310 that encloses an interior region 312. The interior region includes a proximal fluid chamber 314 and a distal fluid chamber 316, separated by a distal insert 318. A plurality of mapping electrodes 320 may be disposed in the external wall 310. A cooling lumen 322 extends from a fluid source (not shown) to the distal fluid chamber 316, and provides fluid to the proximal and distal fluid chambers 314 and 316. As shown, for example, the cooling lumen 322 includes holes 324 to enable a portion of the fluid that is provided to the cooling lumen 322 to pass into the proximal fluid chamber 314 to cool a proximal portion 326 of the ablation electrode 308. The fluid from the proximal chamber 314 also is passed out of the ablation electrode 308 via the proximal irrigation ports 304. The remainder of the fluid provided to the cooling lumen 322 passes to the distal fluid chamber 316 and at least a portion of that fluid is passed out of the ablation electrode 308 via the distal irrigation ports 302.

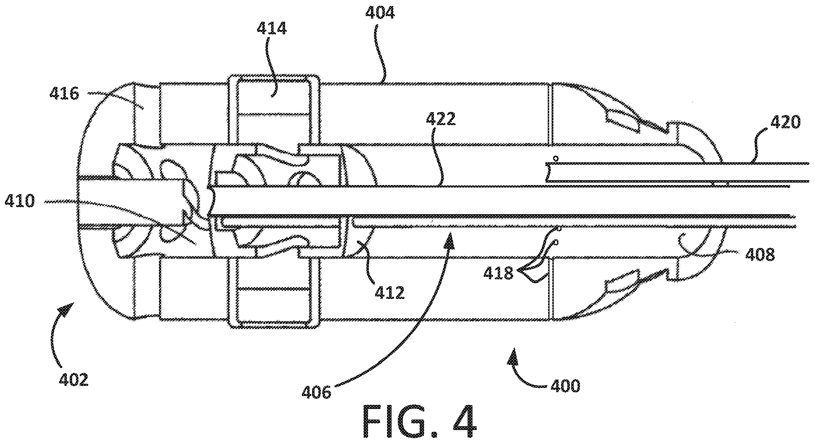

According to embodiments, a catheter may include a separate cooling lumen for providing fluid to each of the proximal and distal fluid chambers. In embodiments, both cooling lumens may be coupled to the same fluid source and/or a separate fluid source. FIG. 4 illustrates a perspective cutaway view of a hybrid catheter tip assembly 400, according to embodiments of the invention, having an ablation electrode 402. The ablation electrode 402 includes an external wall 404 that encloses an interior region 406. The interior region 406 includes a proximal fluid chamber 408 and a distal fluid chamber 410, separated by a distal insert 412. A plurality of mapping electrodes 414 may be disposed in the external wall 404. The external wall 404 may also include a plurality of distal irrigation ports 416 and a plurality of proximal irrigation ports 418. A first cooling lumen 420 extends from a fluid source (not shown) to the proximal fluid chamber 408, and provides fluid to the proximal fluid chamber 418. A second cooling lumen 422 extends from a fluid source (not shown) to the distal fluid chamber 410, and provides fluid to the distal fluid chamber 410. The fluid from the proximal chamber 408 is passed out of the ablation electrode 402 via the proximal irrigation ports 418 and the fluid from the distal fluid chamber 410 is passed out of the ablation electrode 402 via the distal irrigation ports 416.

Electrical signals, such as electrocardiograms (ECGs), are used during a cardiac ablation procedure to distinguish viable tissue from not viable tissue. If ECG amplitudes are seen to attenuate during the delivery of RF energy into the tissue, the delivery of RF energy into that specific tissue may be stopped. However, noise on the ECG signals makes it difficult to view attenuation. It is currently believed that internal cooling fluid circulation, cooling fluid circulating externally in contact with other electrodes, and/or fluid seepage in between the electrodes and their housing may cause the noise on this type of ablation catheter.

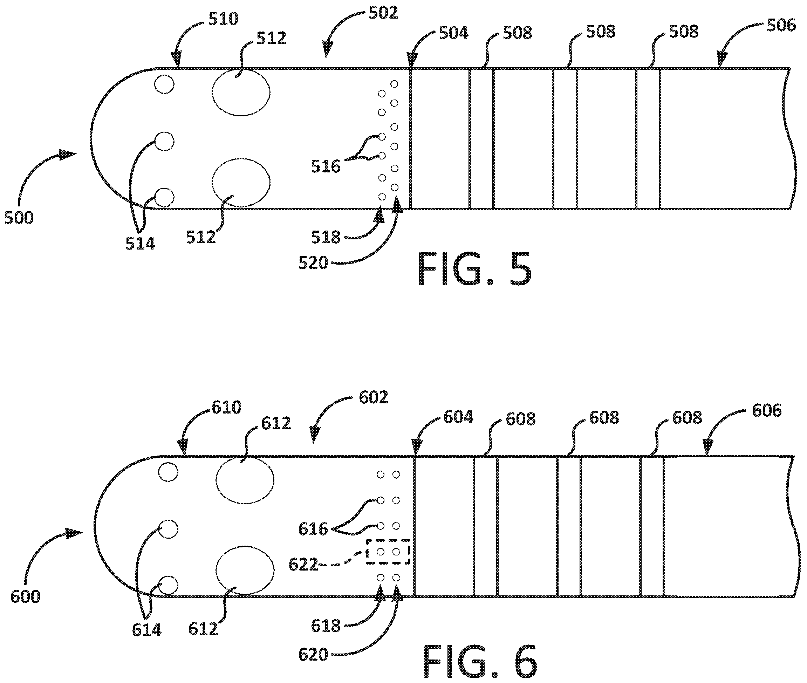

FIG. 5 depicts a hybrid catheter 500 in accordance with embodiments of the invention. The catheter 500 may be, include, or be similar to, the catheter 102 depicted in FIG. 1, the catheter 200 depicted in FIGS. 2A-2D, the catheter 300 depicted in FIGS. 3A and 3B, and/or the catheter tip assembly 400 depicted in FIG. 4. The illustrated catheter 500 includes a tip assembly 502 coupled to a distal end 504 of a catheter body 506. The catheter body 506 includes a plurality of ring electrodes 508. In embodiments, the catheter body 506 may include three ring electrodes 508 or any other desirable number of ring electrodes 508. As illustrated, the tip assembly 502 includes an ablation electrode 510 having a plurality of mapping electrodes 512, a plurality of distal irrigation ports 514, and a plurality of proximal irrigation ports 516. The proximal irrigation ports 516 are arranged in a first row 518 and a second row 520. Each row 518 and 520 may include a plurality of proximal irrigation ports 516 evenly spaced circumferentially around the ablation tip electrode 510. In embodiments, the ablation electrode 510 may include one row of proximal irrigation ports 516, two rows of proximal irrigation ports 516, three rows of proximal irrigation ports 516, four rows of proximal irrigation ports 516, or any other desired number of rows, each row having any number of proximal irrigation ports that may, for example, be evenly spaced circumferentially.

The proximal irrigation ports 516 depicted in FIG. 5, and described above, may be arranged in multiple rows, where each row is offset from an adjacent row. According to embodiments, proximal irrigation ports may be arranged in circumferential rows that are aligned to form multiple longitudinal columns of at least two irrigation ports. FIG. 6 depicts a hybrid catheter 600 in accordance with embodiments of the invention. The catheter 600 may be, include, or be similar to, the catheter 500 depicted in FIG. 5. The illustrated catheter 600 includes a tip assembly 602 coupled to a distal end 604 of a catheter body 606. The catheter body 606 includes a plurality of ring electrodes 608. In embodiments, the catheter body 606 may include three ring electrodes 608 or any other desirable number of ring electrodes 608. As illustrated, the tip assembly 602 includes an ablation electrode 610 having a plurality of mapping electrodes 612, a plurality of distal irrigation ports 614, and a plurality of proximal irrigation ports 616. The proximal irrigation ports 616 are arranged in a first row 618 and a second row 620. Each row 618 and 620 may include a plurality of proximal irrigation ports 616 evenly spaced circumferentially around the ablation electrode 610. The proximal irrigation ports 616 may be arranged in circumferential rows 618 and 620 that are aligned to form multiple longitudinal columns 622 of at least two irrigation ports 616 each. The ablation electrode 610 may include any desired number of rows and/or columns of proximal irrigation ports 616.

Various modifications and additions can be made to the exemplary embodiments discussed without departing from the scope of the present invention. For example, while the embodiments described above refer to particular features, the scope of this invention also includes embodiments having different combinations of features and embodiments that do not include all of the described features. Accordingly, the scope of the present invention is intended to embrace all such alternatives, modifications, and variations as fall within the scope of the claims, together with all equivalents thereof.

## Claims

1. An open-irrigated catheter system (200), comprising:
a catheter body;
a tip assembly (202), coupled to a distal end of the catheter body, and having an exterior wall (218) that defines an interior region within the tip assembly (202), wherein the exterior wall (218) includes a plurality of proximal irrigation ports (244) and a plurality of distal irrigation ports (240), wherein each of the proximal irrigation ports has a diameter of between 0.00254 cm and 0.01016 cm (0.001 in and 0.004 in), and each of the distal irrigation ports has a diameter of between 0.0254 cm to 0.0508 cm (0.01 in to 0.02 in), and wherein the exterior wall is conductive for delivering radio frequency (RF) energy for an RF ablation procedure;
at least one fluid chamber (212) defined within the interior region, wherein the at least one fluid chamber (212) is in fluid communication with at least one of the plurality of proximal irrigation ports (244) and the plurality of distal irrigation ports (240); and
at least one fluid lumen extending from a fluid source, through the catheter body, to the tip assembly, wherein the at least one fluid lumen is in fluid communication with the at least one fluid chamber (212).

2. The system of claim 1, wherein the plurality of proximal irrigation ports (212) comprises at least 12 irrigation ports.

3. The system of claim 2, wherein the plurality of proximal irrigation ports (212) comprises at least 36 irrigation ports.

4. The system of any of claims 1-3, wherein the plurality of proximal irrigation ports (212) is arranged in an evenly-spaced array positioned circumferentially around a proximal portion (238) of the tip assembly.

5. The system of claim 4, wherein the array comprises at least one row.

6. The system of claim 5, the array comprising a first row (518) of irrigation ports and a second row (520) of irrigation ports, wherein the second row is aligned with the first row to form a plurality of pairs of longitudinally-aligned irrigation ports.

7. The system of claim 5, the array comprising a first row of irrigation ports and a second row of irrigation ports, wherein the second row is offset from the first row.

8. The system of any of claims 1-7, wherein the at least one fluid chamber comprises a proximal fluid chamber (212) and a distal fluid chamber (214), wherein the proximal fluid chamber is in fluid communication with the plurality of proximal irrigation ports, and wherein the distal fluid chamber is in fluid communication with the plurality of distal irrigation ports.

9. The system of claim 8, wherein the at least one fluid lumen comprises a first fluid lumen and a second fluid lumen, wherein the first fluid lumen is in fluid communication with the proximal fluid chamber and wherein the second fluid lumen is in fluid communication with the distal fluid chamber.

10. The system of claim 9, comprising a fluid source, wherein the fluid source is configured to provides a first stream of fluid to the proximal fluid chamber and a second stream of fluid to the distal fluid chamber, and wherein the fluid source is further configured to provide the second stream of fluid at a greater flow rate than the first stream of fluid.

11. The system of claim 8, wherein the at least one fluid lumen comprises a single fluid lumen that is in fluid communication with the proximal fluid chamber, the system further comprising a fluid path extending between the proximal fluid chamber and the distal fluid chamber such that the proximal fluid chamber is in fluid communication with the distal fluid chamber.

12. The system of claim 11, further comprising a distal insert, wherein the fluid path is defined within the distal insert (210).

13. The system of any of claims 1-12, wherein the external wall includes a plurality of mapping-electrode openings, and wherein the system further comprises a plurality of mapping electrodes, wherein each of the plurality of mapping electrodes is positioned within one of the plurality of mapping-electrode openings.

14. The system of any of claims 1-13, wherein the plurality of distal irrigation ports comprises six distal irrigation ports, wherein the six distal irrigation ports are evenly-spaced circumferentially around a distal portion of the tip assembly.

## Patentansprüche

1. Offen berieseltes Kathetersystem (200), welches aufweist:
einen Katheterkörper;
eine Spitzenanordnung (202), die mit einem distalen Ende des Katheterkörpers gekoppelt ist und eine äußere Wand (218), die einen inneren Bereich innerhalb der Spitzenanordnung (202) definiert, hat, wobei die äußere Wand (218) mehrere proximale Berieselungsöffnungen (244) und mehrere distale Berieselungsöffnungen (240) enthält, wobei jede der proximalen Berieselungsöffnungen einen Durchmesser zwischen 0,00254 cm und 0,01016 cm (0,001 Zoll und 0,004 Zoll) hat und jede der distalen Berieselungsöffnungen einen Durchmesser zwischen 0,0254 cm bis 0,0508 cm (0,01 Zoll bis 0,02 Zoll) hat, und wobei die äußere Wand leitend ist für die Zuführung von Hochfrequenzenergie (HF-Energie) für einen HF-Ablationsvorgang;
zumindest eine Fluidkammer (212), die innerhalb des inneren Bereichs definiert ist, wobei die zumindest eine Fluidkammer (212) in Fluidverbindung mit zumindest einer der mehreren proximalen Berieselungsöffnungen (244) und der mehreren distalen Berieselungsöffnungen (240) ist; und
zumindest ein Fluidlumen, das sich von einer Fluidquelle durch den Katheterkörper zu der Spitzenanordnung erstreckt, wobei das zumindest eine Fluidlumen in Fluidverbindung mit der zumindest einen Fluidkammer (212) ist.

2. System nach Anspruch 1, bei dem die mehreren proximalen Berieselungsöffnungen (212) zumindest 12 Berieselungsöffnungen aufweisen.

3. System nach Anspruch 2, bei dem die mehreren proximalen Berieselungsöffnungen (212) zumindest 36 Berieselungsöffnungen aufweisen.

4. System nach einem der Ansprüche 1-3, bei dem die mehreren proximalen Berieselungsöffnungen (212) in einer Anordnung mit gleichen Abständen angeordnet sind, die in Umfangsrichtung um einen proximalen Bereich (238) der Spitzenanordnung herum positioniert ist.

5. System nach Anspruch 4, bei dem die Anordnung zumindest eine Reihe aufweist.

6. System nach Anspruch 5, bei dem die Anordnung eine erste Reihe (518) von Berieselungsöffnungen und eine zweite Reihe (520) von Berieselungsöffnungen aufweist, wobei die zweite Reihe mit der ersten Reihe ausgerichtet ist, um mehrere Paare von in Längsrichtung ausgerichteten Berieselungsöffnungen zu bilden.

7. System nach Anspruch 5, bei dem die Anordnung eine erste Reihe von Berieselungsöffnungen und eine zweite Reihe von Berieselungsöffnungen aufweist, wobei die zweite Reihe gegenüber der ersten Reihe versetzt ist.

8. System nach einem der Ansprüche 1-7, bei dem die zumindest eine Fluidkammer eine proximale Fluidkammer (212) und eine distale Fluidkammer (214) aufweist, wobei die proximale Fluidkammer in Fluidverbindung mit den mehreren proximalen Berieselungsöffnungen ist, und wobei die distale Fluidkammer in Fluidverbindung mit den mehreren distalen Berieselungsöffnungen ist.

9. System nach Anspruch 8, bei dem das zumindest eine Fluidlumen ein erstes Fluidlumen und ein zweites Fluidlumen aufweist, wobei das erste Fluidlumen in Fluidverbindung mit der proximalen Fluidkammer ist, und wobei das zweite Fluidlumen in Fluidverbindung mit der distalen Fluidkammer ist.

10. System nach Anspruch 9, aufweisend eine Fluidquelle, wobei die Fluidquelle konfiguriert ist, einen ersten Fluidstrom zu der proximalen Fluidkammer und einen zweiten Fluidstrom zu der distalen Fluidkammer zu liefern, und wobei die Fluidquelle weiterhin konfiguriert ist, den zweiten Fluidstrom mit einer größeren Strömungsgeschwindigkeit als den ersten Fluidstrom zu liefern.

11. System nach Anspruch 8, bei dem das zumindest eine Fluidlumen ein einziges Fluidlumen aufweist, das in Fluidverbindung mit der proximalen Fluidkammer ist, wobei das System weiterhin einen Fluidpfad aufweist, der sich zwischen der proximalen Fluidkammer und der distalen Fluidkammer derart erstreckt, dass die proximale Fluidkammer in Fluidverbindung mit der distalen Fluidkammer ist.

12. System nach Anspruch 11, weiterhin aufweisend einen distalen Einsatz, wobei der Fluidpfad innerhalb des distalen Einsatzes (210) definiert ist.

13. System nach einem der Ansprüche 1-12, bei dem die äußere Wand mehrere Abbildungselektrodenöffnungen enthält, wobei das System weiterhin mehrere Abbildungselektroden aufweist, und jede der mehreren Abbildungselektroden innerhalb einer der mehreren Abbildungselektrodenöffnungen positioniert ist.

14. System nach einem der Ansprüche 1-13, bei dem die mehreren distalen Berieselungsöffnungen sechs distale Berieselungsöffnungen aufweisen, wobei die sechs distalen Berieselungsöffnungen in gleichem gegenseitigem Abstand in Umfangsrichtung um einen distalen Bereich der Spitzenanordnung herum angeordnet sind.

## Revendications

1. Système de cathéter irrigué ouvert (200), comprenant:
un corps de cathéter ;
un assemblage d'extrémité formant embout (202), qui est couplé à une extrémité distale du corps de cathéter, et qui comporte une paroi extérieure (218) qui définit une région intérieure à l'intérieur de l'assemblage d'extrémité formant embout (202), dans lequel la paroi extérieure (218) inclut une pluralité d'orifices d'irrigation proximaux (244) et une pluralité d'orifices d'irrigation distaux (240), dans lequel chacun des orifices d'irrigation proximaux présente un diamètre qui se situe entre 0,00254 cm et 0,01016 cm (0,001 pouce et 0,004 pouce), et chacun des orifices d'irrigation distaux présente un diamètre qui se situe entre 0,0254 cm et 0,0508 cm (0,01 pouce et 0,02 pouce), et dans lequel la paroi extérieure est conductrice pour délivrer une énergie radiofréquence (RF) pour une procédure d'ablation RF ;
au moins une chambre de fluide (212) qui est définie à l'intérieur de la région intérieure, dans lequel l'au moins une chambre de fluide (212) est en communication en termes de fluide avec au moins l'un de la pluralité d'orifices d'irrigation proximaux (244) et de la pluralité d'orifices d'irrigation distaux (240) ; et
au moins une lumière de fluide qui s'étend depuis une source de fluide, au travers du corps de cathéter jusqu'à l'assemblage d'extrémité formant embout, dans lequel l'au moins une lumière de fluide est en communication en termes de fluide avec l'au moins une chambre de fluide (212).

2. Système selon la revendication 1, dans lequel la pluralité d'orifices d'irrigation proximaux (212) comprend au moins 12 orifices d'irrigation.

3. Système selon la revendication 2, dans lequel la pluralité d'orifices d'irrigation proximaux (212) comprend au moins 36 orifices d'irrigation

4. Système selon l'une quelconque des revendications 1 à 3, dans lequel les orifices de la pluralité d'orifices d'irrigation proximaux (212) sont agencés selon un réseau espacé de manière régulière qui est positionné de façon circonférentielle le long d'une section proximale (238) de l'assemblage d'extrémité formant embout.

5. Système selon la revendication 4, dans lequel le réseau comprend au moins une rangée.

6. Système selon la revendication 5, le réseau comprenant une première rangée (518) d'orifices d'irrigation et une seconde rangée (520) d'orifices d'irrigation, dans lequel la seconde rangée est alignée avec la première rangée de manière à former une pluralité de paires d'orifices d'irrigation alignés longitudinalement.

7. Système selon la revendication 5, le réseau comprenant une première rangée d'orifices d'irrigation et une seconde rangée d'orifices d'irrigation, dans lequel la seconde rangée est décalée par rapport à la première rangée.

8. Système selon l'une quelconque des revendications 1 à 7, dans lequel l'au moins une chambre de fluide comprend une chambre de fluide proximale (212) et une chambre de fluide distale (214), dans lequel la chambre de fluide proximale est en communication en termes de fluide avec la pluralité d'orifices d'irrigation proximaux, et dans lequel la chambre de fluide distale est en communication en termes de fluide avec la pluralité d'orifices d'irrigation distaux.

9. Système selon la revendication 8, dans lequel l'au moins une lumière de fluide comprend une première lumière de fluide et une seconde lumière de fluide, dans lequel la première lumière de fluide est en communication en termes de fluide avec la chambre de fluide proximale et dans lequel la seconde lumière de fluide est en communication en termes de fluide avec la chambre de fluide distale.

10. Système selon la revendication 9, comprenant une source de fluide, dans lequel la source de fluide est configurée de manière à ce qu'elle fournisse un premier flux d'écoulement de fluide sur la chambre de fluide proximale et un second flux d'écoulement de fluide sur la chambre de fluide distale, dans lequel la source de fluide est en outre configurée de manière à ce qu'elle fournisse le second flux d'écoulement de fluide à un débit d'écoulement plus important que celui du premier flux d'écoulement de fluide.

11. Système selon la revendication 8, dans lequel l'au moins une lumière de fluide comprend une unique lumière de fluide qui est en communication en termes de fluide avec la chambre de fluide proximale, le système comprenant en outre une voie de fluide qui s'étend entre la chambre de fluide proximale et la chambre de fluide distale de telle sorte que la chambre de fluide proximale soit en communication en termes de fluide avec la chambre de fluide distale.

12. Système selon la revendication 11, comprenant en outre un insert distal, dans lequel la voie de fluide est définie à l'intérieur de l'insert distal (210).

13. Système selon l'une quelconque des revendications 1 à 12, dans lequel la paroi externe inclut une pluralité d'ouvertures d'électrode de cartographie, et dans lequel le système comprend en outre une pluralité d'électrodes de cartographie, dans lequel chacune de la pluralité d'électrodes de cartographie est positionnée à l'intérieur de l'une de la pluralité d'ouvertures d'électrode de cartographie.

14. Système selon l'une quelconque des revendications 1 à 13, dans lequel la pluralité d'orifices d'irrigation distaux comprend six orifices d'irrigation distaux, dans lequel les six orifices d'irrigation distaux sont espacés de manière régulière de façon circonférentielle le long d'une section distale de l'assemblage d'extrémité formant embout.
